Europäisches Patentamt

European Patent Office

Office européen des brevets

(11) Numéro de publication: **0 317 388 B1**

## FASCICULE DE BREVET EUROPEEN

(45) Date de publication de fascicule du brevet: **30.12.92**

(51) Int. Cl.5: **B05B 7/00**, A45D 34/00, A61L 9/14, A61M 11/06

(21) Numéro de dépôt: **88402800.2**

(22) Date de dépôt: **07.11.88**

(54) **Dispositif permettant la diffusion de produits fluides.**

(30) Priorité: **13.11.87 FR 8715715**

(43) Date de publication de la demande:
    **24.05.89 Bulletin 89/21**

(45) Mention de la délivrance du brevet:
    **30.12.92 Bulletin 92/53**

(84) Etats contractants désignés:
    **AT BE CH DE ES FR GB GR IT LI LU NL SE**

(56) Documents cités:
    **DE-C- 579 769**
    **FR-A- 606 998**
    **FR-A- 825 469**
    **FR-A- 1 552 806**
    **US-A- 3 027 097**

(73) Titulaire: **Giusti, Max**
    **Zone Industrielle Ouartier Saint-Joseph**
    **F-04100 Manosque(FR)**

(72) Inventeur: **Garnier, Didier**
    **5, Boulevard Gabriel Banon**
    **F-04700 Oraison(FR)**
    Inventeur: **Giusti, Max**
    **Chemin de Puyimayon**
    **F-04100 Manosque(FR)**

(74) Mandataire: **Rochet, Michel**
    **ELF ATOCHEM S.A. Département Propriété**
    **Industrielle 4-8, Cours Michelet La Défense**
    **10 - Cedex 42**
    **F-92091 Paris-La-Défense(FR)**

EP 0 317 388 B1

## Description

L'invention est relative à un dispositif permettant la diffusion de produits fluides tels que essences, huiles, parfums.

Dans le brevet français FR-A-825469, on a proposé un dispositif de vaporisation comprenant des gicleurs ou ajutages, servant à atomiser ou pulvériser des liquides aqueux ou analogues à vaporiser, spécialement des liquides de type désinfectants ou revivifiant l'air. Selon ce brevet, le fluide à diffuser est contenu dans un récipient (7) et, sous l'effet d'un fluide d'entraînement 20, le fluide à diffuser est entraîné par la combinaison d'une perforation 13 et d'un canal 16 plongeant dans le fluide à diffuser. Un tel vaporisateur, de par sa conception, n'est pas utilisable en fonctionnement intermittent, dans la mesure où, lors de l'arrêt du fluide d'entraînement du liquide à vaporiser, il existe un risque certain de retrouver du liquide dans le système d'entraînement, avec les problèmes de corrosion et autres détériorations dudit système liés au retour du liquide.

L'invention a donc pour objet un dispositif de diffusion de produits fluides comportant une buse dont la géométrie et le mode de fonctionnement empêchent le retour du produit à diffuser à l'intérieur du système d'entraînement dudit produit et préviennent ainsi les détériorations pouvant être occasionnées par ces produits lors d'un fonctionnement intermittent.

Elle concerne également un dispositif qui associe le système anti-retour mentionné ci-avant à un système de régulation de la mise en marche et de l'arrêt de la diffusion du produit fluide, en fonction de la durée de vie des particules de produit diffusé dans leur environnement. Elle concerne également un dispositif dans lequel la fréquence de mise en route et d'arrêt de la diffusion du produit est prédéterminée, ledit dispositif renfermant un module de commande électronique ainsi pré-réglé.

Elle concerne également un dispositif comportant en lui-même un module de commande électronique auto-régulé.

L'invention a donc pour objet un dispositif de diffusion de produits fluides, comprenant un conteneur (7) pour le produit à diffuser, un système d'entraînement (E) dudit fluide à travers une buse (5) et un système de commande (c) dudit système d'entraînement, ladite buse (5) comprenant un canal central (5b) relié à un générateur de fluide d'entraînement et au moins un canal latéral (6a), disposé parallèlement au canal central (5b), plongeant à une extrémité dans le fluide à diffuser et se terminant à l'autre extrémité au niveau d'une perforation (15), le dit dispositif étant caractérisé en ce que, dans le but de permettre la diffusion temporisée des produits fluides sans retour desdits produits dans le système d'entraînement (E) lors de l'arrêt du dispositif, le canal central (5b) est entouré d'une tête (5a) formant essentiellement sur la partie de la longueur du canal central disposée au-dessus du niveau de la perforation (15) et avec la paroi dudit canal central (5b) une cavité (14) destinée au passage du fluide d'entraînement lorsque le dispositif fonctionne et interdisant le retour du produit liquide dans le système d'entraînement lors de l'arrêt du dispositif, la ou les perforations (15) étant disposées sur la partie inférieure de ladite cavité (14), le système de commande (c) comprenant des moyens de réglage de la fréquence de mise en route et d'arrêt dudit dispositif.

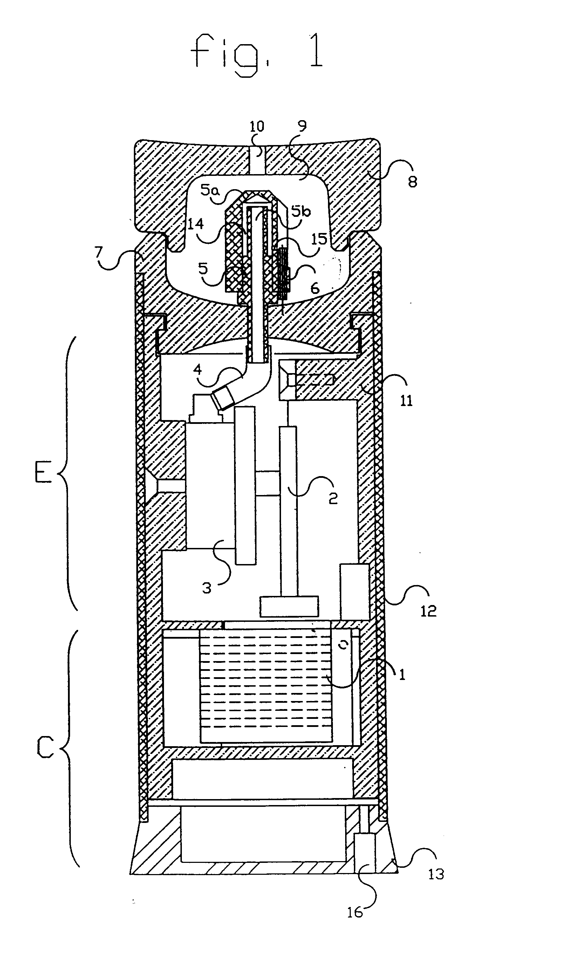

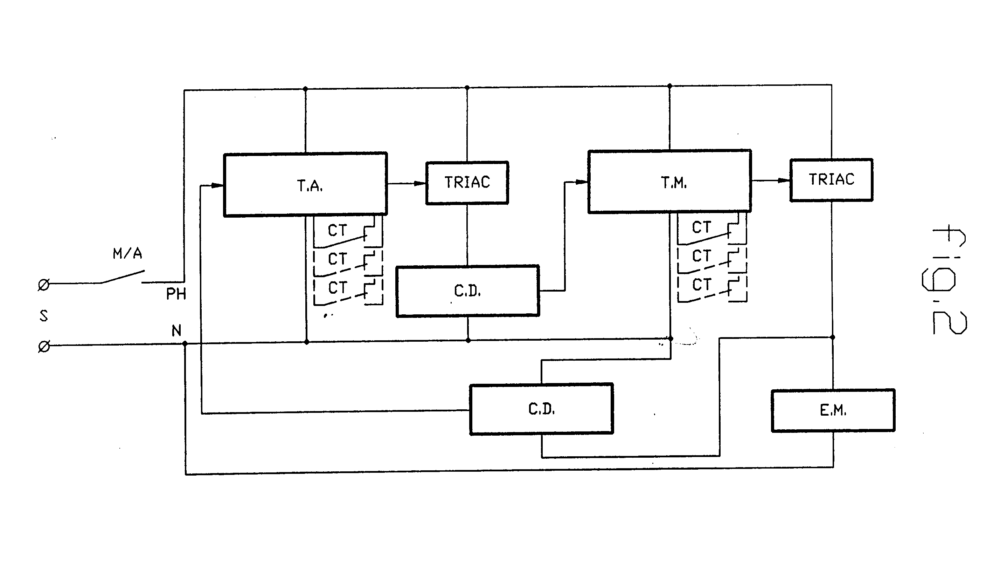

D'autres avantages du dispositif conforme à l'invention apparaîtront à la lecture de l'exposé en liaison avec les figures 1 et 2 qui suit.

D'une manière préférentielle l'extrémité du canal ou des canaux latéraux (6) au regard de la ou des perforations (15) est taillée en biseau. Avantageusement, l'angle formé par le plan du biseau avec l'axe de la perforation (15) est compris entre 30° et 80°.

Le ou les canaux latéraux (6) peuvent avantageusement être logés partiellement dans la tête (5a) et aboutir sensiblement au niveau des perforations (15) à la base d'une partie évidée, formée dans ladite tête dans le prolongement desdits canaux (6).

Le ou les canaux latéraux (6) peuvent être rendus solidaires, par exemple par collage, de la tête (5a) ou du canal central (5b), ou encore du conteneur (7) ou même de la coiffe (8) dont il sera question plus loin.

Dans ce dispositif le système d'entraînement du fluide à diffuser (E) comprend un générateur de fluide d'entraînement avantageusement constitué par une pompe (3) reliée à une source de fluide d'entraînement, un organe de liaison (4) avec le tube central (5b), la pression délivrée par la pompe et les dimensionnements de l'organe de liaison avec le tube central étant réglés pour établir une dépression dans le ou les tubes latéraux (6) suffisante pour entraîner le fluide à diffuser.

La dépression ainsi réalisée provoque l'aspiration du fluide à diffuser à la base du conteneur (7) par le canal latéral (6). La colonne de fluide ainsi formée est cassée au niveau de l'extrémité du canal (6) correspondant à la perforation (15) et notamment lorsque l'extrémité dudit canal est taillée en biseau, provoquant ainsi l'éclatement des particules et leur diffusion. Selon une modalité avantageuse, cette diffusion s'effectue dans un premier temps dans une chambre de préparation (9) formée en plaçant une coiffe (8) au-dessus du conteneur (7) et autour de la buse (5) avant d'être éjectée par au moins un orifice (10) prévu à la surface de cette coiffe. L'emplacement de l'orifice ou de plusieurs orifices et leurs dimensions peuvent être déterminés par l'homme du métier, compte tenu notamment de la dimension des particules de fluide diffusé et de leur aptitude à être diffusées, inhalées ou à se déposer.

Selon une modalité avantageuse, la coiffe (8) se présente sous forme sensiblement cylindrique ouverte à l'extrémité correspondant au conteneur et dont l'autre extrémité est constituée par une surface bi-concave, perforée en son centre, ladite forme bi-concave permettant d'orienter la diffusion du fluide et en même temps de recycler le fluide diffusé et ayant pu se redéposer sur ladite coiffe.

La présence de la cavité (14) dans la buse conforme à l'invention permet de piéger le produit fluide pendant le temps d'un arrêt du système d'entraînement, empêchant ainsi le retour du fluide dans le système d'entraînement et le risque de détérioration dudit système.

La tête (5a) peut être solidarisée avec le canal central (5b) par exemple par vissage ou par collage.

Le fluide d'entraînement du fluide à diffuser est avantageusement constitué par l'air environnant le dispositif. L'air peut pénétrer dans le dispositif par exemple par un orifice (16) situé à la base (13) du dispositif et en fonction de l'état dudit air environnant, il peut être avantageux de faire passer cet air à travers un dispositif de filtration (non représenté) avant d'alimenter le générateur de fluide d'entraînement (E). Cet air peut également être séché et/ou réchauffé.

Le dispositif comprend également un système de commande (C) du système d'entraînement du fluide.

Ce système de commande comprend des moyens de mise en route et d'arrêt du générateur de fluide d'entraînement. Plus précisément ce système comprend des moyens de réglage de la fréquence de mise en route et d'arrêt du générateur en fonction de la concentration du fluide diffusé dans le milieu environnant.

Ces moyens de réglage sont avantageusement constitués par un système électronique de temporisation de phases d'arrêt du système et de phases de fonctionnement dudit système, plusieurs phases d'arrêt et/ou plusieurs phases de fonctionnement pouvant avoir des durées différentes. Ce même système électronique permet également de choisir des valeurs variables du débit du fluide à diffuser. De ce fait on pourra choisir des phases de marche de durée différentes, à des débits variables, entrecoupées de phases d'arrêt également de durée différentes, toutes les combinaisons durée/débit étant possibles et déterminables en fonction du fluide à diffuser et notamment de sa densité, de la pression de projection et du volume dans lequel le fluide est diffusé.

L'alimentation du dispositif peut se faire par branchement électrique simple, sur 110/220 volts alternatifs, 50/60 hertz ou sous basse tension, la mise sous tension ou l'arrêt définitif pouvant être commandés par un simple interrupteur ou par un système de télécommande, qui peut également déclencher les diverses temporisations.

La mise en marche et l'arrêt du système d'entraînement peut être commandée par le système électronique par exemple par déclenchement d'un électro-aimant (1) commandant directement ou par l'intermédiaire d'un bras (2) le générateur de fluide entraîneur (3).

Le système électronique de réglage de la fréquence des phases mise en marche/arrêt et, éventuellement du débit de fluide diffusé peut être pré-réglé avant sa mise en place dans le dispositif, compte-tenu de la nature du fluide à diffuser et de l'environnement dans lequel il sera placé. La figure 2 présente un schéma de branchement utilisable. Sur cette figure, les symboles ont les significations suivantes : TA = temporisation arrêt ; TM = temporisation marche ; CD = circuit de déclenchement ; TRIAC = interrupteur statique ; CT = connection de temporisation ; EM = électro-aimant ; M/A = marche/arrêt ; PH = phase ; N = neutre ; S = secteur.

Les connections de temporisation correspondent à des valeurs différentes de temporisation, aussi bien à la marche qu'à l'arrêt.

Ce système peut également comporter une pluralité de possibilités de temporisation des phases marche/arrêt et éventuellement de valeurs de débit et être commandé par l'introduction dans le dispositif d'instructions spécifiques, par exemple au moyen de cartes perforées ou de cartes magnétiques correspondant aux couples fluide à diffuser/environnement.

Selon une autre variante, le système électronique de réglage est auto-régulé. A cette fin, ce système comprend des moyens d'entrée d'informations concernant au moins le liquide à diffuser et le volume dans lequel ce liquide sera diffusé, des moyens d'analyse desdites informations et de détermination de la concentration du fluide dans cet environnement et des moyens de déclenchement du système d'entraînement en fonction de ladite concentration.

Le dispositif conforme à l'invention est avantageusement contenu dans un ensemble enveloppe (12), corps isolant (11) permettant une sécurité par double isolement.

Ce dispositif permet la diffusion de fluides divers tels que huiles essentielles, produits d'agrément à base de parfums, de désodorisants, substances médicamenteuses, insecticides, cette liste n'étant donnée qu'à titre purement indicatif.

Ce dispositif est particulièrement intéressant par son aptitude à diffuser une quantité de produit adaptée au milieu dans lequel il sera diffusé. En outre, de par sa construction, il met à l'abri des risques de détérioration du dispositif du fait du retour du produit à diffuser au moment de l'arrêt du système d'entraînement. De ce fait il convient notamment pour la diffusion de substances pouvant appartenir aux familles mentionnées plus haut, et se montrant particulièrement agressives vis à vis des matériaux généralement utilisés dans la fabrication de dispositifs de diffusion, tels que métaux, matières plastiques, caoutchouc.

## Revendications

1. Dispositif de diffusion de produits fluides, comprenant un conteneur (7) pour le produit à diffuser, un système d'entraînement (E) dudit fluide à travers une buse (5) et un système de commande (C) dudit système d'entraînement, ladite buse (5) comprenant un canal central (5b) relié à un générateur de fluide d'entraînement et au moins un canal latéral (6a), disposé parallèlement au canal central (5b), plongeant à une extrémité dans le fluide à diffuser et se terminant à l'autre extrémité au niveau d'une perforation (15), le dit dispositif étant caractérisé en ce que, dans le but de permettre la diffusion temporisée des produits fluides sans retour desdits produits dans le système d'entraînement (E) lors de l'arrêt du dispositif, le canal central (5b) est entouré d'une tête (5a) formant essentiellement sur la partie de la longueur du canal central disposée au-dessus du niveau de la perforation (15) et avec la paroi dudit canal central (5b) une cavité (14) destinée au passage du fluide d'entraînement lorsque le dispositif fonctionne et interdisant le retour du produit liquide dans le système d'entraînement lors de l'arrêt du dispositif, la ou les perforations (15) étant disposées sur la partie inférieure de ladite cavité (14), le système de commande (C) comprenant des moyens de réglage de la fréquence de mise en route et d'arrêt dudit dispositif.

2. Dispositif selon la revendication 1, caractérisé en ce que l'extrémité du canal ou des canaux latéraux (6) au regard de la ou des perforations (15) est taillée en biseau, l'angle formé par le plan dudit biseau avec l'axe de la perforation (15) étant compris entre 30° et 80°.

3. Dispositif selon l'une quelconque des revendications 1 et 2, caractérisé en ce que le canal latéral (6) est logé partiellement dans la tête (5a) et aboutit sensiblement au niveau de la perforation (15), à la base d'une partie évidée formée dans ladite tête dans le prolongement dudit canal (6).

4. Dispositif selon l'une quelconque des revendications 1 à 3, caractérisé en ce que le canal latéral (6) est solidaire de la tête (5a).

5. Dispositif selon l'une quelconque des revendications 1 à 3, caractérisé en ce que le canal latéral (6) est solidaire du canal central (5b).

6. Dispositif selon l'une quelconque des revendications 1 à 3, caractérisé en ce que le canal latéral (6) est solidaire du conteneur (7).

7. Dispositif selon l'une quelconque des revendications 1 à 6, caractérisé en ce que le système d'entraînement du fluide à diffuser (E) comprend un générateur de fluide d'entraînement pouvant être constitué par une pompe (3) reliée à une source de fluide d'entraînement, un organe de liaison avec le tube central (5b), la pression délivrée par le générateur et les dimensionnements de l'organe de liaison avec le tube central étant réglés pour établir une dépression dans le ou les tubes latéraux (6) suffisante pour entraîner le fluide à diffuser.

8. Dispositif selon l'une quelconque des revendications 1 à 7, caractérisé en ce que l'ensemble conteneur (7) et buse (5) est couvert par une coiffe (8) formant chambre de préparation (9) de la diffusion, ladite coiffe comportant au moins un orifice (10) permettant ladite diffusion.

9. Dispositif selon l'une quelconque des revendications 1 à 8, caractérisé en ce que le fluide d'entraînement du fluide à diffuser est constitué par l'air environnant le dispositif.

10. Dispositif selon la revendication 9, caractérisé en ce que l'air environnant est filtré et/ou réchauffé avant de traverser le générateur du fluide d'entraînement.

11. Dispositif selon la revendication 10, caractérisé en ce que le système de commande (C) comprend des moyens de réglage de la fréquence de mise en route et d'arrêt du générateur en fonction de la concentration du fluide diffusé dans le milieu environnant.

12. Dispositif selon la revendication 11, caractérisé en ce que les moyens de réglage comprennent au moins une temporisation de l'état de marche et une temporisation de l'état d'arrêt.

**13.** Dispositif selon l'une quelconque des revendications 10 à 12, caractérisé en ce que les moyens de réglage ont également une action sur le débit de diffusion du liquide.

**14.** Dispositif selon l'une quelconque des revendications 10 à 13, caractérisé en ce que les moyens de réglage sont préréglés.

**15.** Dispositif selon l'une quelconque des revendications 10 à 13, caractérisé en ce que les moyens de réglage du dispositif comportent une pluralité de valeurs de temporisation et éventuellement de débit, le choix d'une ou plusieurs desdites valeurs étant provoqué par l'introduction dans le dispositif d'instructions spécifiques.

**16.** Dispositif selon la revendication 15, caractérisé en ce que l'introduction d'instructions spécifiques se fait au moyen de cartes perforées ou de cartes magnétiques.

**17.** Dispositif selon l'une quelconque des revendications 10 à 13, caractérisé en ce que les moyens de réglage sont auto-régulés et sont constitués par un système électronique comprenant des moyens d'entrée d'informations concernant au moins le liquide' à diffuser et le volume dans lequel ce liquide sera diffusé, des moyens d'analyse desdites informations et de détermination de la concentration du liquide diffusé dans cet environnement et des moyens de déclenchement du système d'entraînement en fonction de ladite concentration.

**18.** Dispositif selon l'une quelconque des revendications 11 ou 12, caractérisé en ce que les informations concernant le liquide à diffuser comprennent au moins la densité et la pression de projection dudit liquide

**19.** Application du dispositif selon l'une quelconque des revendications 1 à 18 à la diffusion d'huiles essentielles, de parfums, de désodorisant, d'insecticides, de substances médicamenteuses.

**Claims**

**1.** Device for diffusing fluid products comprising a container (7) for the product to be diffused, a system (E) for propelling the said product through a nozzle (5) and a system (C) for controlling the said propellant system, the said nozzle (5) comprising a central duct (5b) connected to a generator of the propellant fluid and at least one lateral duct (6a) disposed parallel to the central duct (5b), dipping at one end into the fluid to be diffused and terminating at the other end at the level of a perforation (15), the said device being characterized in that with a view to permitting the timed diffusion of the fluid products without any return of the said products into the propellant system (E) when the device is stopped, the central duct (5b) is surrounded by a head (5a) forming mainly over the part of the length of the central duct disposed above the level of the perforation (15) and together with the wall of the said central duct (5b), a cavity (14) intended for the passing of the propellant fluid when the device is operating and inhibiting the return of the liquid product into the propellant system when the device is stopped, the perforation or perforations (15) being disposed over the lower portion of the said cavity (14), the control system (C) comprising means for regulating the frequency of starting and stopping the said device.

**2.** Device according to claim 1, characterized in that the end of the lateral duct or ducts (6) opposite the perforation or perforations (15) has a bevel cut, the angle formed by the plane of the said bevel with the axis of the perforation (15) being comprised between 30° and 80°.

**3.** Device according to any one of claims 1 and 2, characterized in that the lateral duct (6) is partly accommodated in the head (5a) and terminates substantially at the level of the perforation (15), at the base of a cut out portion formed in the said head in the extension of the said duct (6).

**4.** Device according to any one of claims 1 to 3, characterized in that the lateral duct (6) is joined to the head (5a).

**5.** Device according to any one of claims 1 to 3, characterized in that the lateral duct (6) is joined to the central duct (5b).

**6.** Device according to any one of claims 1 to 3, characterized in that the lateral duct (6) is joined to the container (7).

**7.** Device according to any one of claims 1 to 6, characterized in that the system (E) for propelling the fluid to be diffused comprises a generator of a propellant fluid which may be constituted by a pump (3) connected to a source of a propellant fluid, an element for connection to the central tube (5b), the pressure delivered by the generator and the dimensioning of the element for connection to the central tube being regulated so as to establish a low pressure in the lateral tube or tubes (6) that is sufficient for propelling the fluid to be diffused.

8. Device according to any one of claims 1 to 7, characterized in that the set of the container (7) and nozzle (5) is covered by a cap (8) forming a chamber (9) for preparing the diffusion, the said cap comprising at least one orifice (10) permitting the said diffusion.

9. Device according to any one of claims 1 to 8, characterized in that the fluid for propelling the fluid to be diffused is constituted by the air surrounding the device.

10. Device according to claim 9, characterized in that the surrounding air is filtered and/or reheated before passing through the generator of the propellant fluid.

11. Device according to claim 10, characterized in that the control system (C) comprises means for regulating the frequency of starting and stopping the generator according to the concentration of the diffused fluid in the ambient environment.

12. Device according to claim 11, characterized in that the regulating means include at least one timing for the operating state and one timing for the stopped state.

13. Device according to any one of claims 10 to 12, characterized in that the regulating means also act on the diffusion rate of the liquid.

14. Device according to any one of claims 10 to 13, characterized in that the regulating means are preset.

15. Device according to any one of claims 10 to 13, characterized in that the means for regulating the device include a plurality of values for the timing and for the rate of delivery, if required, the choice of one or several of the said values being produced by introducing specific instructions into the device.

16. Device according to claim 15, characterized in that the introduction of specific instructions is effected by means of punched cards or magnetic cards.

17. Device according to any one of claims 10 to 13, characterized in that the regulating means are self-regulating and are constituted by an electronic system comprising means for entering the data concerning at least the liquid to be diffused and the space wherein this liquid will be diffused, means for analysing the said data and for determining the concentration of the diffused liquid in this environment, and means for starting the propellant system according to the said concentration.

18. Device according to any one of claims 11 or 12, characterized in that the data concerning the liquid to be diffused include at least the density and the ejection pressure of the said liquid.

19. Application of the device according to any one of claims 1 to 18 to the diffusion of essential oils, perfumes, deodorants, insecticides, medicinal substances.

**Patentansprüche**

1. Apparatur zur Zerstäubung flüssiger Produkte, die einen Behälter (7) für das zu zerstäubende Produkt, ein über eine Düse (5) funktionierendes Treibsystem (E) für besagte Flüssigkeit und ein Steuerungssystem (C) für dieses Treibsystem umfaßt, wobei die Düse (5) einen Zentralkanal (5b) enthält, der mit einem Generator für die Treibflüssigkeit und mindestens einem seitlichen Kanal (6a) verbunden ist, der parallel zum Zentralkanal (5b) angeordnet ist und dessen eines Ende in die zu zerstäubende Flüssigkeit eintaucht, während das andere Ende exakt auf der Höhe einer Bohrung (15) endet. Obige Apparatur ist dadurch gekennzeichnet, daß, zum Zweck der zeitlich festgelegten Zerstäubung flüssiger Produkte ohne Zurücklaufen derselben Produkte in das Treibsystem (E) während des Stillstands der Apparatur, der Zentralkanal (5b) von einem Kopf (5a) umschlossen ist, der im wesentlichen mit der Wand des Zentralkanals (5b) über die Teillänge des Zentralkanals, die oberhalb der Bohrung (15) liegt, eine Höhlung (14) bildet, welche der Passage der Treibflüssigkeit während des Betriebs der Apparatur dient, welche aber das Zurücklaufen des flüssigen Produkts in das Treibsystem nach Abschalten der Apparatur verhindert, wobei die Bohrung beziehungsweise die Bohrungen (15) im unteren Teil besagter Höhlung (14) angebracht sind und das Steuerungssystem (C) die Möglichkeit zur Einstellung der An- und Abschaltfrequenz obiger Apparatur beinhaltet.

2. Apparatur nach Anspruch 1, dadurch gekennzeichnet, daß das auf die Bohrung (15) zugehende Ende des seitlichen Kanals (6) beziehungsweise die auf die Bohrungen (15) zugehenden Enden der seitlichen Kanäle (6) über eine Schrägkante spitz zulaufen, wobei der Winkel, der von der Fläche dieser Schrägkante mit der Achse der Bohrung (15) gebildet wird, zwischen 30 und 80° liegt

3. Apparatur nach einem der Ansprüche 1 oder 2, dadurch gekennzeichnet, daß der seitliche Kanal (6) sich teilweise im Kopf (5a) befindet und exakt auf der Höhe der Bohrung (15) endet, am unteren Ende eines Hohlraums, der in besagtem Kopf in der Verlängerung des seitlichen Kanals (6) gebildet wird.

4. Apparatur nach einem der Ansprüche 1 bis 3, dadurch gekennzeichnet, daß der seitliche Kanal (6) direkt mit dem Kopf (5a) verbunden ist.

5. Apparatur nach einem der Ansprüche 1 bis 3, dadurch gekennzeichnet, daß der seitliche Kanal (6) direkt mit dem Zentralkanal (5b) verbunden ist.

6. Apparatur nach einem der Ansprüche 1 bis 3, dadurch gekennzeichnet, daß der seitliche Kanal (6) direkt mit dem Behälter (7) verbunden ist.

7. Apparatur nach einem der Ansprüche 1 bis 6, dadurch gekennzeichnet, daß das Treibsystem (E) für die zu versprühende Flüssigkeit einen Generator für die Treibflüssigkeit enthält, der aus einer Pumpe (3), welche mit einer Quelle für die Treibflüssigkeit verbunden ist, und einem Verbindungselement (4) mit dem Zentralkanal (5b) bestehen kann, wobei der durch den Generator gelieferte Druck und die Ausmaße des Verbindungselements mit dem Zentralkanal so eingestellt sind, daß der in dem oder den seitliche Kanälen (6) eintretende Druckabfall ausreicht, um die zu zerstäubende Flüssigkeit mitzureißen.

8. Apparatur nach einem der Ansprüche 1 bis 7, dadurch gekennzeichnet, daß der Behälter (7) und die Düse (5) gemeinsam von einer Haube (8) bedeckt sind, so daß eine Präparationskammer (9) für die Zerstäubung gebildet wird, wobei besagte Haube mindestens eine Öffnung (10) besitzt, die diese Zerstäubung ermöglicht.

9. Apparatur nach einem der Ansprüche 1 bis 8, dadurch gekennzeichnet, daß die Treibflüssigkeit für die zu zerstäubende Flüssigkeit aus der Luft gebildet wird, die die Apparatur umgibt.

10. Apparatur nach Anspruch 9, dadurch gekennzeichnet, daß die Luft aus der Umgebung filtriert und/oder erwärmt wird, bevor sie den Generator für die Treibflüssigkeit passiert.

11. Apparatur nach Anspruch 10, dadurch gekennzeichnet, daß das Steuerungssystem (C) die Möglichkeit beinhaltet, die An- und Abschaltfrequenz des Generators in Abhängigkeit von der Konzentration der zerstäubten Flüssigkeit in der Umgebung einzustellen.

12. Apparatur nach Anspruch 11, dadurch gekennzeichnet, daß die Regulierungsmöglichkeiten zumindest die Einstellung je einer Dauer für den Zerstäubungsvorgang und für den Stillstand der Apparatur umfassen.

13. Apparatur nach einem der Ansprüche 10 bis 12, dadurch gekennzeichnet, daß die Regulierungsmöglichkeiten auch die Einstellung eines bestimmten Durchsatzes an zerstäubter Flüssigkeit beinhalten.

14. Apparatur nach einem der Ansprüche 10 bis 13, dadurch gekennzeichnet, daß die Regulierungseinheit voreingestellt ist.

15. Apparatur nach einem der Ansprüche 10 bis 13, dadurch gekennzeichnet, daß die Regulierungsmöglichkeiten der Apparatur eine Vielzahl von Einstellungen zeitlich festgelegter Funktionen und eventuell des Durchsatzes umfassen, wobei die Wahl einer oder mehrerer dieser Einstellungen durch die Eingabe spezifischer Anweisungen hervorgerufen wird.

16. Apparatur nach Anspruch 15, dadurch gekennzeichnet, daß die Eingabe spezifischer Anweisungen durch Lochkarten oder Magnetkarten geschieht.

17. Apparatur nach einem der Ansprüche 10 bis 13, dadurch gekennzeichnet, daß die Regulierungseinheit selbstgesteuert ist und aus einem elektronischen System besteht, das die Möglichkeit zur Eingabe von Informationen zumindest bezüglich der zu zerstäubenden Flüssigkeit und des Volumens, in dem diese Flüssigkeit zerstäubt werden wird, besitzt ebenso wie die Möglichkeit zur Analyse besagter Informationen und zur Bestimmung der Konzentration der zerstäubten Flüssigkeit in dieser Umgebung und die Möglichkeit zur Auslösung des Treibsystems in Abhängigkeit von besagter Konzentration.

18. Apparatur nach einem der Ansprüche 11 oder 12, dadurch gekennzeichnet, daß die Informationen, die die zu zerstäubende Flüssigkeit betreffen, zumindest die Dichte und den Schleuderdruck besagter Flüssigkeit umfassen.

19. Anwendung der Apparatur nach einem der Ansprüche 1 bis 18 zur Zerstäubung etherischer Öle, von Parfums, von Desodoranzien, von Insektiziden und medikamentösen Substanzen.

fig. 1

# fig.2